# DEMANDE DE BREVET EUROPEEN

(11) **EP 3 338 774 A1**
(43) Date de publication de la demande: **27.06.2018**
(21) Numéro de dépôt: 16306811.7
(22) Date de dépôt: 23.12.2016
(51) Int. Cl.: A61K 31/255, A23K 50/80, A61P 33/00, A61P 31/04

(54) **COMPOSITION À BASE DE COMPOSÉS THIOSULFINATE ET/OU DE THIOSULFONATE POUR SON UTILISATION DANS LA PRÉVENTION DES INFECTIONS BACTÉRIENNES CHEZ LES ANIMAUX AQUATIQUES**

(71) Demandeur: MIXSCIENCE, 35170 Bruz (FR)
(72) Inventeur: FROUEL, Stéphane, 35000 RENNES (FR); JOZWIAK, Frédéric, 56380 GUER (FR); BANCHEREAU, Thi, 44700 ORVAULT (FR)
(74) Mandataire: Monni, Richard

(57) **Abrégé**

L'invention concerne une composition comprenant au moins un composé de formule I suivante R-SOa-S-R' (I), dans lequel a est égal à 1 ou 2 et R et R' sont indépendamment l'un de l'autre un groupe alkyl en C1 - C5, linéaire ou branché, pour une utilisation dans le cadre de la prévention des infections parasitaires et notamment bactériennes des animaux aquatiques, ledit au moins un composé susmentionné représentant 0,003 % en poids par rapport au poids total de la composition

## Description

L'invention concerne une composition alimentaire et ses utilisations, notamment dans le domaine de l'aquaculture.

Le terme "aquaculture" est utilisé pour qualifier l'élevage d'organismes aquatiques (poissons, algues, crustacés, mollusques...) en milieu fermé (bassin, rivière, étang, cage). Pratiqué depuis plusieurs milliers d'années en Asie, ce système de "domestication" des ressources aquatiques connaît un essor considérable à l'échelle mondiale et est devenu une importante activité économique dans de nombreux pays.

Aujourd'hui, le challenge le plus important de cette industrie est d'optimiser le rendement de production afin de satisfaire la demande de plus en plus grandissante du consommateur. Les principaux axes de développement pour atteindre cet objectif consistent à augmenter l'intensification et la diversification des espèces aquacoles. Dans les unités de production à grande échelle, où les animaux aquatiques sont exposés à des conditions de stress, les problèmes liés aux maladies et à la détérioration des conditions environnementales sont de plus en plus fréquents et engendrent de sérieuses pertes économiques.

La prévention et le contrôle des infections reposent principalement sur l'utilisation de produits vétérinaires. Jusqu'à présent, le contrôle des problèmes bactériens, était basé sur l'utilisation d'antibiotiques. Leur emploi massif a favorisé l'émergence de souches bactériennes résistantes. Une telle résistance peut être facilement transférée à d'autres souches, soit en suivant des modifications du génome déjà présent, soit par transfert de matériel génétique entre cellules par l'intermédiaire de plasmides ou de bactériophages. Des résistances dues au transfert de plasmides ont été reconnues chez de nombreux pathogènes bactériens incluant les genres *Aeromonas spp, Edwardsiella spp, Photobacterium spp, Streptococcus spp, Tenacibaculum spp, Vibrio spp, Yersinia spp...* Pour limiter les problèmes de résistance liés aux antibiotiques, des traitements réellement adaptés à chaque espèce et des procédés alternatifs doivent être développés pour maintenir un environnement microbien sain dans les élevages.

Les alternatives actuellement prometteuses comprennent la vaccination, l'immunostimulation et les probiotiques.

La vaccination est un outil important dans la prévention des maladies infectieuses chez les humains et les animaux : et elle est largement utilisée chez les poissons. Le terme de vaccin ne devrait être strictement appliqué que lorsque le but est d'obtenir une protection durable par activation de la mémoire immunologique puisqu'il cible la réponse immunitaire spécifique. La vaccination nécessite une stimulation primaire avec un antigène et dépend des lymphocytes à mettre en oeuvre. De plus, la plupart des vaccins commerciaux augmentent habituellement la résistance à seulement un ou deux pathogènes spécifiques et confèrent seulement une résistance temporaire à la maladie ce qui fragilise l'intérêt de cette alternative.

Les immunostimulants, quant à eux, peuvent renforcer l'immunité contre une grande variété d'agents pathogènes : ils sont donc non spécifiques. Les immunostimulations peuvent être obtenues dans un sens plus général, par exemple, en ciblant l'activation du complément, l'activation des macrophages, la phagocytose accrue par des neutrophiles et des monocytes et la sécrétion de cytokines, sans nécessiter une réponse spécifique à un antigène défini. Les immunostimulants ont été étudiés de manière approfondie chez les poissons et les crustacés à la fois à l'échelle de l'animal et à l'échelle cellulaire. Ils sont utilisés comme agents prophylactiques pour lutter contre les maladies infectieuses des animaux et jouent également le rôle de molécules d'alarme qui activent le système immunitaire non spécifique. Cependant, l'utilisation de plusieurs immunostimulants pendant des périodes prolongées ne semble pas fournir d'avantages supplémentaires par rapport à une dose unique ; un surdosage peut même inhiber les réponses immunitaires et ainsi diminuer la croissance des animaux. Enfin, leur effet est normalement de courte durée et ils sont souvent inefficaces une fois la maladie présente. Cette alternative est donc également limitée.

Enfin, les probiotiques ont pris depuis quelques années une importance considérable en aquaculture. Ces derniers, sont définis comme étant des microorganismes vivants qui exercent une action bénéfique sur l'hôte en régulant de manière positive sa flore microbienne intestinale, en optimisant son utilisation de l'aliment ingéré, en stimulant sa réponse immunitaire contre les pathogènes et en améliorant la qualité de son environnement ambiant. L'utilisation de probiotiques et de produits commerciaux contenant des probiotiques en aquaculture (souvent en élevages de crevettes) a montré des résultats de résistance aux maladies similaires à ceux obtenus avec des antibiotiques couramment utilisés. Ils sont donc une alternative intéressante pour résoudre le problème des antibiorésistances. Cependant, par définition, les probiotiques sont utilisés en tant qu'agents prophylactiques. Ils peuvent prévenir de l'apparition d'une maladie mais ne constituent pas une stratégie curative efficace puisqu'ils agissent sur l'hôte plus que sur le pathogène. En outre, en tant que microorganismes vivants, quand ils sont appliqués, aucun autre traitement chimique destiné à lutter contre les microorganismes tels que les pathogènes fongiques ou parasitaires ne peut être utilisé sous réserve d'altérer l'efficacité des probiotiques. Aussi, ces derniers peuvent être facilement détruits par des composés chimiques ou des médicaments ce qui interfère avec leur efficacité et leur intérêt à long terme.

Ces méthodes alternatives, bien qu'ayant démontré leur efficacité, sont limitées par le fait qu'elles sont essentiellement préventives, souvent spécifiques d'un ou de quelques pathogènes, et sont peu efficaces dans le traitement curatif à long terme d'une maladie. Elles dépendent également de la physiologie de l'animal hôte et plusieurs facteurs tels que la génétique de l'espèce, le stade biologique et l'environnement d'élevage interagissent avec le type et le dosage de ces produits ce qui peut diminuer leur efficacité.

Le développement d'actifs naturels aux propriétés antimicrobiennes offre une efficacité moins dépendante de l'environnement et de la physiologie de l'hôte, et un plus large spectre antimicrobien puisque ces actifs ciblent directement le pathogène ce qui autorise les 2 types d'applications : prophylactique au niveau de l'hôte et thérapeutique au niveau de l'hôte et de l'environnement d'élevage.

On connaît ainsi de l'état de la technique le propyl thiosulfinate (PTS) et le propyl thiosulfonate (PTSO), dérivés naturels d'alliacés comme composés anti-bactériens et anti-microbiens à large spectre affectant aussi bien les microorganismes unicellulaires telles que les bactéries gram positif, les bactéries gram négatif et les virus ou d'autres pathogènes comme les parasites pluricellulaires.

Par exemple, la demande EP 2552432 A2 propose d'utiliser les composés propyl thiosulfinate (PTS) de formule R-SOa-S-R où R représente un groupe n-propyl (-CH2-CH2-CH3) et propyl thiosulfonate (PTSO) de formule R-SOa-S-R où R représente un groupe n-propyl (-CH2-CH2-CH3) pour réduire le nombre de pathogènes apicomplexes chez un animal. En l'occurrence, un apicomplexe est défini comme étant un organisme unicellulaire, parasite des vertébrés et des invertébrés. Le terme apicomplexe regroupe les genres *Babesia, Cryptosporidium, Eimeria, Gregarina, Monospora, Neospora, Plasmodium, Theileri* et *Toxoplasma*. La composition décrite dans ce document est essentiellement utilisée dans le cadre d'élevage de la volaille, du porc ou des ruminants mais n'inclut pas les espèces aquatiques.

La demande internationale WO 2015049587 A2 propose d'utiliser les mêmes composés pour réduire les parasites pathogènes chez les animaux aquatiques. En l'occurrence, les parasites étudiés regroupent les parasites appartenant à différents groupes zoologiques tels que les dinoflagellés ectoparasites, les ciliés, les zooflagellés, les helminthes tels que les monogènes, les trématodes, les cestodes, les nématodes, les parasites ectoparasites tels que les mollusques, les crustacés et les poux de mer. Toutefois, ce document ne s'intéresse pas à la contamination bactérienne et virale de bassins aquacoles.

Aussi, le traitement des infections des structures et du milieu d'élevage demeurent.

L'invention a pour objectif de remédier à ce manque.

Un des buts de l'invention est de fournir une nouvelle composition adaptée à la problématique d'aquaculture.

L'invention a également pour but de proposer un moyen efficace et facilement utilisable de traiter ou de prévenir les infections parasitaires rencontrées dans le cadre des cultures aquacoles.

L'invention concerne une composition comprenant au moins un composé de formule I suivante

R-SOa-S-R' (I)

dans lequel a est égal à 1 ou 2 et R et R' sont indépendamment l'un de l'autre un groupe alkyl en C1 - C5, linéaire ou branché, aliphatique ou non,
pour une utilisation quotidienne ou plus régulière, pendant au moins huit jours, dans le cadre de la prévention des infections parasitaires, et notamment bactériennes ou virales, des animaux aquatiques élevés dans des structures aquacoles, notamment en bassins d'aquaculture
ledit au moins un composé susmentionné représentant 0,003 % en poids par rapport au poids total de la composition.

De manière avantageuse, l'invention concerne une composition comprenant au moins un composé de formule I suivante

R-SOa-S-R' (I)

dans lequel a est égal à 1 ou 2 et R et R' sont indépendamment l'un de l'autre un groupe alkyl en C1 - C5, linéaire ou branché, aliphatique ou non,
et au moins un support minéral ledit au moins un composé représentant en poids 15 % par rapport au poids du mélange dudit au moins un composé et dudit au moins un support minéral,
pour une utilisation quotidienne ou plus régulière, pendant au moins huit jours, dans le cadre de la prévention des infections parasitaires, et notamment bactériennes ou virales, des animaux aquatiques élevés dans des structures aquacoles,
ledit au moins un composé susmentionné représentant 0,003 % en poids par rapport au poids total de la composition.

L'invention repose sur la constatation faite par les inventeurs qu'une dose particulière d'au moins un composé de formule I, dans un mode d'administration précis, permet de prévenir l'infection par des bactéries des structures aquacoles. Les inventeurs ont montré que la dose de 0,003% était particulièrement adaptée à la prévention des infections bactériennes.

Par « utilisation quotidienne ou plus régulière » on entend dans l'invention une utilisation une fois par jour, voire plusieurs fois par jour, par exemple matin et soir, ou matin, midi et soir. Cette utilisation dépendra de l'habitude de l'éleveur et des besoins alimentaires des animaux aquatiques envisagés. L'homme du métier saura adapter la fréquence selon l'espèce.

Par « pendant au moins huit jours » on entend dans l'invention une utilisation de 8 jours, 9 jours, 10 jours, 11 jours, 12 jours, 13 jours, 14 jours, 15 jours, 16 jours, 17 jours, 18 jours, 19 jours, 20 jours, 21 jours, 22 jours, 23 jours, 24 jours, 25 jours, 26 jours, 27 jours, 28 jours, 29 jours, 30 jours ou 31 jours, voire plus. L'homme du métier adaptera l'utilisation selon les besoin prophylactiques de la culture aquacole considérée. En tout état de cause, il est important que l'utilisation soit d'au moins 8 jours.

Les bactéries aquatiques, cibles de la composition selon l'invention, dans le cadre de l'utilisation susmentionnée sont les bactéries des genres suivants : *Aerococcus, Aeromonas, Allivibrio, Brucella, Campylobacter, Carnobacterium, Chryseobacterium,* Citrobacter, Clostridium, Corynebacterium, Edwardsiella, Elizabethkingia, Escherichia, *Eubacterium, Flavobacterium, Francissella, Hafnia, Halomonas, Lactococcus,* Listonella, Moraxella, Moritella, Mycobacterium, Neisseria, Nocardia, Pasteurella, *Photobacterium, Plesiomonas, Pseudomonas, Renibacterium, Rhodococcus, Salmonella, Serratia, Shewanella, Staphylococcus, Streptobacillus, Streptococcus, Tenacibaculum, Vagococcus, Vibrio* et *Yersinia*.

Les virus aquatiques, cibles de la composition selon l'invention, dans le cadre de l'utilisation susmentionnée sont les virus des genres suivants ainsi que les genres viraux suivants : *Alphavirus, Aquabirnavirus, Baculovirus, Betanodavirus, Brevidensovirus, virus Cricket paralysis, Densovirus, Ebolavirus, Giardiavirus, Isavirus, Nodavirus, Novirhabdovirus, Okavirus, Orthomyxovirus, Ranavirus, Rhabdovirus, Totivirus* et *Whispovirus*.

De manière encore plus avantageuse, l'invention concerne une composition comprenant au moins deux composés de formule I suivante

R-SOa-S-R' (I)

dans lequel a est égal à 1 ou 2 et R et R' sont indépendamment l'un de l'autre un groupe alkyl en C1 - C5, linéaire ou branché, aliphatique ou non, pour l'utilisation susmentionnée,
lesdits au moins deux composés étant de structure chimique différente.

En d'autres termes, l'invention concerne la composition pour son utilisation susmentionnée, ladite composition comprenant aux moins deux, notamment deux, composés de formule I distincte, lesdits au moins deux composés représentant 0,003% en poids par rapport au poids total de la composition.

Il peut être particulièrement avantageux que les deux composés, qui ont une structure chimique distincte, soient tels que le premier ait a=1 et le deuxième composé ait a=2. En tout état de cause, les deux composés ne peuvent en aucun cas être de formule chimique identique, ou ne se différencier l'un de l'autre que par une forme saline.

Avantageusement, l'invention concerne la composition pour son utilisation susmentionnée, dans laquelle ladite composition comprend un mélange des composés suivants

CH₃-CH₂-CH₂-SO-S-CH₂-CH₂-CH₃ et CH₃-CH₂-CH₂-SO₂-S-CH₂-CH₂-CH₃

ledit mélange représentant en poids 0,003% de ladite composition.

Dans l'invention, la molécule de formule CH₃-CH₂-CH₂-SO-S-CH₂-CH₂-CH₃ est appelée thiosulfinate de di (n-propyl) ou PTS, et la molécule de formule CH₃-CH₂-CH₂-SO₂-S-CH₂-CH₂-CH₃ est appelée thiosulfonate de di (n-propyl) ou PTSO.

Dans un mode de réalisation plus avantageux, l'invention concerne une composition comprenant un mélange de PTS et de PTSO, pour l'utilisation susmentionnée.

Il est également avantageux que la composition susmentionnée soit sous la forme d'une poudre qui peut être soit dispersée, par exemple dans l'eau d'élevage, soit incluse dans un aliment, ou encore dispersée sur ou dans un aliment.

En particulier, il est avantageux que la composition susmentionnée comprenne au moins un composé, notamment au moins deux composés susmentionnés, et un support minéral, ledit au moins un composé représentant en poids 15 % par rapport au poids du mélange dudit au moins un composé et dudit au moins un support minéral.

De manière encore plus avantageuse, la composition susmentionnée est telle qu'elle comprend :
a. 20% en poids du mélange dudit au moins un composé et dudit au moins un support minéral,
b. de 40% à 61 % en poids par rapport au poids total de la composition d'un ou plusieurs composés permettant la stabilité et l'hydro dispersion de la composition, et de 19% à 40 % en poids d'un ou plusieurs composés immunostimulants.

Une composition particulièrement avantageuse de l'invention, est telle qu'elle comprend les ingrédients suivants :
20% en poids d'un mélange constitué de 15% en poids d'un mélange PTS/PTSO et 85% en poids d'un support de silice,
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice.

Une composition particulièrement avantageuse de l'invention est telle qu'elle comprend les ingrédients suivants :
20% en poids d'un mélange constitué de 15% en poids d'un mélange PTS/PTSO et 85% en poids d'un support de silice,
34% en poids de bentonite
17% en poids de sépiolite
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice.

Aussi, l'un des aspects particulièrement avantageux de l'invention concerne une composition constituée de
20% en poids d'un mélange constitué de 15% en poids d'un mélange PTS/PTSO et 85% en poids d'un support de silice,
34% en poids de bentonite
17% en poids de sépiolite
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice,
pour une utilisation quotidienne ou plus régulière, ou plus fréquente, dans un complément alimentaire, pendant au moins huit jours, dans le cadre de la prévention des infections parasitaires, et notamment bactériennes, des animaux aquatiques élevés en bassins d'aquaculture,
ledit mélange PTS/PTSO étant utilisé à 0,003 % en poids par rapport au poids total du complément alimentaire.

La composition susmentionnée est telle qu'elle rend le produit stable, facilement utilisable en aquaculture, hydro-dispersible ayant avant tout une activité antimicrobienne, renforcée par des propriétés immunostimulantes et protectrices du système cellulaire des animaux aquatiques cibles.

En résumé, un des aspects avantageux est d'utiliser une composition susmentionnée à raison de 0.1% de la composition totale (comprenant le principe actif de formule I et les autres composés). Puisque avantageusement, les composés actifs de la composition (PTS/PTSO) représentent 15% d'une fraction de 20% en poids par rapport au poids total de la composition, on comprendra donc que le principe actif (mélange PTS/PTSO) est utilisé à 0,003% en poids par rapport total de la composition initiale.

En d'autres termes, l'invention concerne avantageusement une composition comprenant au moins un composé de formule I suivante

R-SOa-S-R' (I)

dans lequel a est égal à 1 ou 2 et R et R' sont indépendamment l'un de l'autre un groupe alkyl en C1 - C5, linéaire ou branché, aliphatique ou non,
ladite composition étant utilisée diluée, suspendue ou dispersée au 1000^{ème}, dans un solvant ou un solide, de manière quotidienne ou plus régulière, pendant au moins huit jours, dans le cadre de la prévention des infections parasitaires, et notamment bactériennes ou virales, des animaux aquatiques élevés dans des structures aquacoles,
de sorte que ledit au moins un composé susmentionné représente, lors de l'utilisation diluée, suspendue ou dispersée au 1000^{ème}, 0,003 % en poids par rapport au poids total de la composition initiale non diluée, suspendue ou dispersée.

Encore plus avantageusement, l'invention concerne la composition pour son utilisation susmentionnée, dans laquelle lesdits animaux de bassins d'aquaculture sont les poissons ou les crustacés décapodes.

Des exemples de poissons et de crustacés décapodes, notamment les crevettes, sont donnés, sans pour autant être limitatifs, dans les exemples ci-après.

Encore plus avantageusement, l'invention concerne la composition susmentionnée pour son utilisation susmentionnée, dans laquelle les dits au moins deux composés sont dans des proportions relatives en poids variant de 60/40 à 40/60.

On entend donc dans l'invention un mélange de PTS/PTSO ou le PTS représente 60% du mélange avec le PTSO alors que le PTSO représente 40% de ce mélange, et inversement. Est également couvert par l'invention un mélange comprenant 50% de chacun des deux composés.

Aussi, dans l'invention, par proportions relatives de 60/40 à 40/60 on entend les proportions PTS/PTSO suivantes : 60 /40, 59 /41, 58 /42, 57 /43, 56 /44, 55 /45, 54 /46, 53 /47, 52 /48, 51 /49, 50 /50, 49 /51, 48 /52, 47 /53, 46 /54, 45 /55, 44 /56, 43 /57, 42 /58, 41 /59, et 40 /60.

L'invention concerne en outre une composition comprenant au moins un composé de formule I suivante

R-SOa-S-R' (I)

dans lequel a est égal à 1 ou 2 et R et R' sont indépendamment l'un de l'autre un groupe alkyl en C₁C₅, linéaire ou branché, aliphatique ou non,
pour une utilisation quotidienne ou plus régulière, pendant au moins huit jours, dans le cadre du traitement des infections parasitaires, et notamment bactériennes, des animaux aquatiques élevés dans des structures aquacoles, notamment en bassins d'aquaculture,
ledit au moins un composé susmentionné représentant de 0,009 à 0,012 % en poids par rapport au poids total de la composition.

Dans l'invention, on entend par « de 0,009 à 0,012 % en poids » des proportions de 0,009%, 0,0091%, 0,0092%, 0,0093%, 0.0094%, 0,0095%, 0,0096%, 0,0097%, 0,0098%, 0,0099%, 0,01%, 0,0101%, 0,0102%, 0,0103%, 0.0104%, 0,0105%, 0,0106%, 0,0107%, 0,0108%, 0,0109%, 0,0110%, 0,0111%, 0,0112%, 0,0113%, 0.0114%, 0,0115%, 0,0116%, 0,0117%, 0,0118%, 0,0119%, 0,012% en poids.

Avantageusement, l'invention concerne la composition pour son utilisation susmentionnée, ladite composition comprenant aux moins deux, notamment deux, composés de formule I, ladite composition comprenant 0,009 à 0,012 % en poids desdits composés, notamment deux composés, par rapport au poids total de la composition, ladite composition comprenant notamment un mélange des composés suivants :

CH₃-CH₂-CH₂-SO-S-CH₂-CH₂-CH₃ et CH₃-CH₂-CH₂-SO₂-S-CH₂-CH₂-CH₃, notamment dans des proportions 60:40 à 40:60 en poids l'un par rapport à l'autre, notamment 50 :50.

Avantageusement, l'invention concerne une composition constituée de
20% en poids d'un mélange constitué de 15% en poids d'un mélange PTS/PTSO et 85% en poids d'un support de silice,
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice,

Avantageusement, l'invention concerne une composition constituée de
20% en poids d'un mélange constitué de 15% en poids d'un mélange PTS/PTSO et 85% en poids d'un support de silice,
34% en poids de bentonite
17% en poids de sépiolite
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice,
pour une utilisation quotidienne ou plus régulière ou fréquente dans un complément alimentaire, pendant au moins huit jours, dans le cadre du traitement des infections parasitaires, et notamment bactériennes, des animaux aquatiques élevés en bassins d'aquaculture,
ledit mélange PTS/PTSO étant utilisé de 0,009 à 0,012 % en poids par rapport au poids total de la composition.

En résumé, un des aspects avantageux est d'utiliser une composition susmentionnée à raison de 0.3 à 0.4% en poids de la composition totale (comprenant le principe active de formule I et les autres composés). Puisque avantageusement, les composés actifs de la composition (PTS/PTSO) représentent 15% d'une fraction de 20% en poids par rapport au poids total de la composition, on comprendra donc que le principe actif (mélange PTS/PTSO) est utilisé de 0,009 à 0,012% en poids par rapport total de la composition initiale.

En d'autres termes, l'invention concerne avantageusement une composition comprenant au moins un composé de formule I suivante

R-SOa-S-R' (I)

dans lequel a est égal à 1 ou 2 et R et R' sont indépendamment l'un de l'autre un groupe alkyl en C1 - C5, linéaire ou branché, aliphatique ou non,
ladite composition étant utilisée diluée, suspendue ou dispersée de 3. 1000^{ème} à 4.1000^{ème}, dans un solvant ou un solide, de manière quotidienne ou plus régulière, pendant au moins huit jours, dans le cadre de la prévention des infections parasitaires, et notamment bactériennes ou virales, des animaux aquatiques élevés en bassins d'aquaculture,
de sorte que ledit au moins un composé susmentionné représente, lors de l'utilisation diluée, suspendue ou dispersée de 3. 1000^{ème} à 4.1000^{ème}, de 0,009 % à 0,012% en poids par rapport au poids total de la composition initiale non diluée, non suspendue ou non dispersée.

En outre, l'invention concerne un produit alimentaire comprenant au moins un nutriment, et la composition telle que définie ci-dessus, pour l'une des utilisations telles que définies ci-dessus.

Le produit alimentaire susmentionné comprend des nutriments nécessaires à la croissance, au maintien du métabolisme basal et à la survie de l'animal auquel il est destiné. Ces nutriments sont les nutriments de base à savoir des glucides, sous forme de sucres simples ou complexes, des lipides, notamment sous forme d'acides gras saturés ou non, de stérols, ou de protides, comprenant notamment tout ou partie des vingt acides aminés. Les nutriments comprennent également des minéraux et des vitamines. Une composition alimentaire selon l'invention est décrite dans les exemples ci-après.

Par ailleurs, l'invention concerne une composition comprenant :
- au moins un composé de formule I suivante :

   R-SOa-S-R' (I)

   dans lequel a est égal à 1 ou 2 et R et R' sont indépendamment l'un de l'autre un groupe alkyl en C1-C5, linéaire ou branché, aliphatique ou non, et
- au moins un support minéral,
   ledit au moins un composé représentant en poids 15 % par rapport au poids du mélange dudit au moins un composé et dudit au moins un support minéral.

De manière encore plus avantageuse, l'invention concerne une composition comprenant
- au moins deux composés de formule I suivante

   R-SOa-S-R' (I)

   dans lequel a est égal à 1 ou 2 et R et R' sont indépendamment l'un de l'autre un groupe alkyl en C1 - C5, linéaire ou branché, aliphatique ou non, pour l'utilisation susmentionnée,
   lesdits au moins deux composés représentant en poids 15 % par rapport au poids du mélange dudit au moins un composé et dudit au moins un support minéral, lesdits au moins deux composés étant de structure chimique différente,
      et
- au moins un support minéral.

Avantageusement, l'invention concerne la composition susmentionnée, comprenant :
a. 20% en poids du mélange dudit au moins un composé, ou au moins deux composés, et dudit au moins un support minéral, dudit au moins un composé, ou au moins deux composés, représentant en poids 15 % par rapport au poids du mélange dudit au moins un composé, ou au moins deux composés, et dudit au moins un support minéral
b. de 40 à 61 % en poids par rapport au poids total de la composition de composés permettant la stabilité et l'hydro dispersion de la composition, et
c. de 19% à 40 % en poids de composés immunostimulants.

Encore plus avantageusement, l'invention concerne une composition constituée de
20% en poids d'un mélange constitué de 15% en poids d'un mélange PTS/PTSO et 85% en poids d'un support de silice,
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice.

Avantageusement, l'invention concerne la composition susmentionnée, comprenant :
a. 20% en poids du mélange dudit au moins un composé et dudit au moins un support minéral,
b. de 40 à 61 % en poids par rapport au poids total de la composition de composés permettant la stabilité et l'hydro dispersion de la composition, et
c. de 19% à 40 % en poids de composés immunostimulants.

Encore plus avantageusement, l'invention concerne une composition constituée de
20% en poids d'un mélange constitué de 15% en poids d'un mélange PTS/PTSO et 85% en poids d'un support de silice,
34% en poids de bentonite
17% en poids de sépiolite
10% en poids de sorbitol
5% en poids de dextrose
5% en poids de vitamine E et 5% en poids de vitamine C, et
4% en poids de méthionine,
les pourcentages étant exprimés en poids par rapport au poids total de la composition à l'exception des pourcentages en poids du mélange PTS/PTSO et support de silice qui sont exprimés en poids par rapport au poids total du mélange PTS/PTSO et support de silice.

En outre, l'invention concerne un produit alimentaire comprenant au moins un nutriment, et la composition telle que définie ci-dessus.

Le produit alimentaire susmentionné comprend des nutriments nécessaires à la croissance, au maintien du métabolisme basal et à la survie de l'animal auquel il est destiné. Ces nutriments sont les nutriments de base à savoir des glucides, sous forme de sucres simples ou complexes, des lipides, notamment sous forme d'acides gras saturés ou non, de stérols, ou de protides, comprenant notamment tout ou partie des vingt acides aminés. Les nutriments comprennent également des minéraux et des vitamines. Une composition alimentaire selon l'invention est décrite dans les exemples ci-après.

L'invention sera mieux comprise à la lumière des exemples et des figures suivantes :

### BREVE DESCRIPTION DES FIGURES

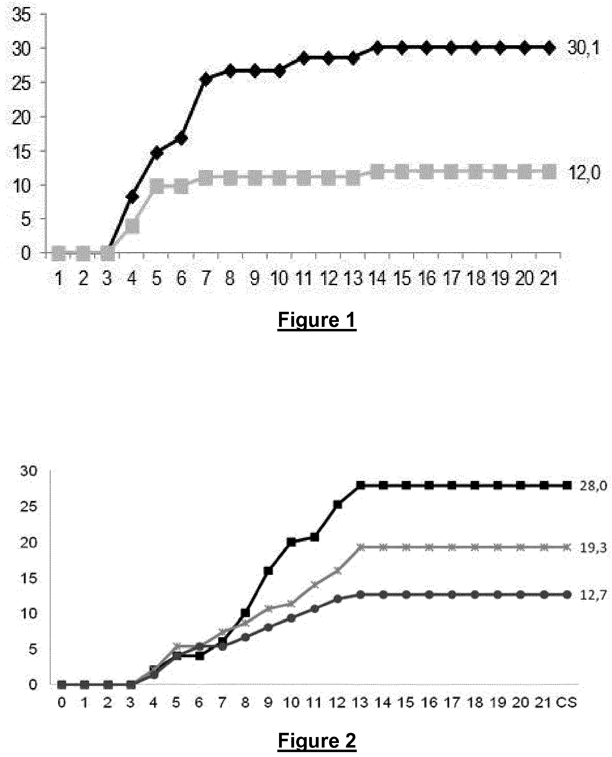

La **Figure 1** représente des courbes qui illustrent la mortalité cumulée (axe des ordonnées) observée en % chez les juvéniles de bar européen *Dicentrarchus labrax,* 21 jours après infection avec *Photobacterium damselae subsp. piscicida* souche PP3 selon que les poissons sont non supplémentés (courbe avec losanges noirs) avec la composition décrite dans l'invention appliquée à 0,1% (soit 0,003% de PTS/PTSO ; courbe avec des carrés gris) pendant 28 jours avant l'infection expérimentale.
La **Figure 2** représente des courbes qui illustrent la mortalité observée chez les juvéniles de tilapia du Nil *Oreochromis niloticus*, 21 jours après infection avec *Streptococcus agalactiae* selon que les poissons sont non supplémentés (courbe avec des carrés noirs) avec la composition décrite dans l'invention appliquée à 0,1% (soit 0,003% de PTS/PTSO ; courbe avec des croix grises) ou que les poissons sont supplémentés avec un antibiotique (courbe avec des ronds) pendant 28 jours avant l'infection expérimentale.

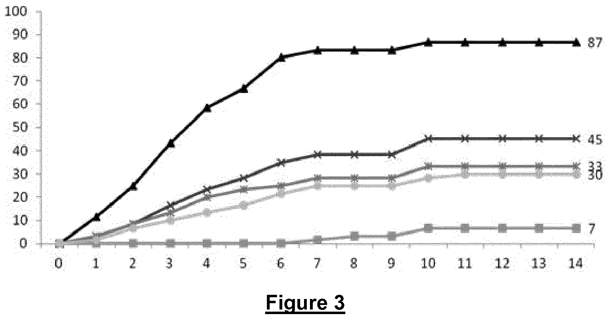

La **Figure 3** représente des courbes qui illustrent la mortalité observée chez les post larves de crevette blanche *Penaeus vannamei* 14 jours après infection avec *Vibrio parahaemolyticus* selon que les crevettes sont non infectées (courbe avec des carrés gris) ou infectées (courbe avec des triangles noirs) ou infectés et supplémentées avec la composition décrite dans l'invention appliquée à 0,1% (courbe avec des croix noires), ou à 0,3% (courbe avec des croix grises) ou à 0,5% (courbe avec des ronds gris), 20 jours avant l'infection expérimentale.

### EXEMPLES

### Exemple 1 - Préparation de la composition préférée de l'invention

La composition décrite dans l'invention repose sur le potentiel antibactérien des composés R-SOa-S-R. Une des versions avantageuse de la composition de l'invention comprend des composés R-SOa-S-R spécifiques mélangés à sept autres ingrédients aux propriétés stabilisatrices et immunostimulantes. La composition est conçue de façon à apporter un produit stable, facilement utilisable en aquaculture, hydro-dispersible ayant avant tout une activité antimicrobienne, renforcée par des propriétés immunostimulantes et protectrices du système cellulaire des animaux aquatiques cibles.

La principale étape de fabrication de la composition consiste à produire les composés R-SOa-S-R. Bien que ces derniers puissent être issus de la synthèse chimique, on peut avantageusement les extraire de plantes appartenant à la famille des alliacées (selon la classification phylogénétique APG) et en particulier au genre Allium, qui est un membre de cette famille. Les plantes les plus connues appartenant au genre Allium sont le poireau (Allium ampeloprasum), l'oignon (Allium cepa L.), l'ail cultivé (Allium sativum L.), l'échalote (Allium ascalonicum), la ciboule (Allium fistulosum L.) et la ciboulette (Allium schoenoprasum L.).

Le PTSO peut être obtenu par synthèse ou décomposition (ou oxydation) du PTS. L'obtention du PTS par extraction et du PTSO est notamment décrite dans la demande de brevet EP A11721534. Les composés R-SOa-S-R peuvent également être obtenus selon le procédé décrit dans la demande de brevet FR A2 813 884.

Une fois obtenus, les composés R-SOa-S-R alors sous forme d'huile sont mélangés à de la silice respectivement selon le rapport en poids 15:85 (%) afin de transformer la solution de composés R-SOa-S-R en poudre.

Cette poudre contenant les composés R-SOa-S-R est ensuite mélangée à l'aide d'une mélangeuse industrielle aux sept ingrédients suivants également sous forme de poudre et selon l'ordre indiqué: bentonite (34%), poudre contenant les composés R-SOa-S-R (20%), sepiolite (17%), sorbitol (10%), dextrose (5%), vitamine E (5%), vitamine C (5%) et méthionine (4%), les pourcentages étant exprimés en poids par rapport au poids total de la composition.

Les composés R-SOa-S-R constituent à ce moment 3% en poids du poids de la composition.

Une application de la composition de 8 ingrédients de 0.1% à 0.4% en poids de l'aliment signifie que les composés R-SOa-S-R constituent 0.003% à 0.012% du poids final de l'aliment.

L'aliment décrit dans l'invention est un aliment destiné à l'alimentation animale et plus précisément à l'alimentation d'animaux aquacoles dans le but de leur fournir les nutriments nécessaires à leur croissance, survie, et métabolisme de base. Le terme d'aliment aquacole a ici le sens normalement attribué par l'homme de métier. Par exemple, un aliment aquacole peut être considéré comme des aliments artificiels (c'est-à-dire formulés et fabriqués) pour suppléer ou remplacer des aliments naturels dans l'industrie de l'aquaculture. Ces aliments sont communément produits sous forme de flocon, de granulé ou de tablette.

Typiquement, un aliment aquacole réfère à un aliment artificiel qui est utile à la croissance des poissons et crustacés de type décapodes. Les aliments formulés sont composés de différents ingrédients dans des proportions variées qui se complètent les uns avec les autres pour former un aliment complet qui répond aux besoins nutritionnels de l'espèce cible aquacole.

Les aliments aquacoles sont composés de micro et de macro ingrédients. En général, les ingrédients, utilisés à des doses supérieures à 1%, sont appelés macro ingrédients. Les ingrédients, utilisés à des doses inférieures à 1% sont appelés micro ingrédients. Chacune des catégories macro et micro ingrédients est subdivisée en composant ayant des fonctions nutritionnelles ou des fonctions techniques. Les ingrédients ayant une fonction technique améliorent les qualités physiques de l'aliment aquacole. Les ingrédients ayant une fonction nutritive apportent aux animaux aquatiques les nutriments et l'énergie nécessaires à leur croissance, à leur survie et à leur métabolisme de base. Ces nutriments sont les nutriments de base à savoir des glucides, sous forme de sucres simples ou complexes, des lipides, notamment sous forme d'acides gras saturés ou non, de stérols, ou de protides, comprenant notamment tout ou partie des vingt acides aminés. Les nutriments comprennent également des minéraux et des vitamines.

La composition peut être incluse à l'aliment pendant les stades larvaires, post larvaires, juvéniles ou tout autre stade de croissance de l'animal aquacole.

En l'absence de maladie, la composition décrite dans l'invention est ajoutée à titre préventif dans l'aliment aquacole et au cours du processus de fabrication de cet aliment à la dose de 0.1 %. L'intégration de la composition, considérée comme micro ingrédient se fait au moment du mélange des ingrédients à la dose recommandée avant le processus d'extrusion ou le processus de presse.

Une fois la maladie déclarée, la composition décrite dans l'invention est ajoutée en curatif dans l'aliment aquacole directement sur les granulés extrudés et à la dose de 0.4%. La quantité de la composition sous forme de poudre, équivalente à 0,4% de l'aliment est diluée dans de l'eau selon un rapport poudre:eau de 1:15 puis pulvérisée directement à la surface de l'aliment grâce à une rampe de pulvérisation. Si besoin, les granulés sont ensuite enrobés avec de l'huile animale ou végétale afin d'éviter le relargage de la composition une fois dans l'eau d'élevage des espèces aquatiques.

### Exemple 2 - Effet in vitro

L'activité antimicrobienne de la composition constituant l'invention est testée par mesure des concentrations inhibitrices minimales exprimées en parties par millions (ppm) vis-à-vis de 10 pathogènes aquatiques bactériens. Les bactéries d'eau douce sont représentées par Aeromonas salmonicida, Edwardsiella tarda et Yersinia ruckeri tandis que les bactéries d'eau de mer ou d'eaux saumâtres sont représentées par Aeromonas salmonicida salmonicida, Photobacterium damsela damsela, Streptococcus iniae, Tenacibaculum maritimum, Tenacibaculum discolor, Vibrio harveyi, Vibrio alginolyticus et Vibrio anguillarum,

La composition (A) constituée de l'ensemble des 8 ingrédients décrits dans l'invention ainsi que 3 produits naturels reconnus comme ayant un potentiel antimicrobien : (B) du citral, constituant majeur de l'huile de citronnelle et d'autres plantes du genre Cymbopogon, (C) de l'eugénol, constituant principal du clou de girofle et (D) du carvacrol, constituant principal de l'huile de thym et d'origan, sont mis en solution dans de l'eau distillée et dilués successivement dans de l'eau distillée directement dans les puits d'une microplaque 96 puits. 100 µL de culture bactérienne cible à la dose de 106 Unités Formant Colonie /mL (UFC/mL) au moment de l'incubation sont ajoutés dans chaque puits de la microplaque. La microplaque est ensuite incubée 18h à 25°C. Les concentrations minimales inhibitrices sont ensuite calculées par observation des puits montrant ou non une croissance bactérienne en fonction de la dose de composition ou de produit associé. Plus la concentration minimale inhibitrice est faible, plus l'efficacité du produit est forte car il faut moins de produit pour induire une inhibition du pathogène.

Après 18h d'incubation, les résultats (tableau 1 ci-après) indiquent que la composition de 8 ingrédients décrite dans l'invention présente les concentrations minimales inhibitrices les plus faibles comprises selon la souche pathogène entre 16 et 125 ppm contre 64 et 1000 ppm pour le produit B, contre 64 et 2000 ppm pour le produit C et contre 32 et 250 ppm pour le produit D. Ces valeurs indiquent que l'efficacité de la composition décrite dans l'invention est la meilleure comparée aux autres produits antibactériens.

**Tableau 1.** Le tableau 1 illustre les concentrations minimales inhibitrices (exprimées en ppm) vis-à-vis de 10 souches pathogènes, de la composition décrite dans l'invention en comparaison avec 3 produits reconnus comme antibactériens. (A) Composition constituée de l'ensemble des 8 ingrédients décrits dans l'invention, (B) Citral, (C) Eugénol et (D) Carvacrol.

| | **(A)** | **(B)** | **(C)** | **(D)** |
|---|---|---|---|---|
| ***Aeromonas salmonicida*** | 64 | 250 | 250 | 250 |
| ***Edwardsiella tarda*** | 125 | 1000 | 500 | 250 |
| ***Photobacterium damsela*** | 32 | 500 | 1000 | 125 |
| ***Streptococcus iniae*** | 125 | 500 | 1000 | 64 |
| ***Tenacibaculum maritimum*** | 16 | 64 | 64 | 32 |
| ***Tenacibaculum discolor*** | 16 | 64 | 125 | 64 |
| ***Vibrio harveyi*** | 64 | 1000 | 1000 | 250 |
| ***Vibrio alginolyticus*** | 125 | 1000 | 2000 | 250 |
| ***Vibrio anguillarum*** | 16 | 500 | 1000 | 64 |
| ***Yersinia ruckeri*** | 125 | 1000 | 500 | 250 |

### Exemple 3 - Comparaison de l'effet du PTS/PTSO et de la composition selon l'invention

Les activités antimicrobiennes de la composition constituant l'invention (A) et du mélange constitué des seuls composés R-SOa-S-R (B) sont comparées à celles d'antibiotiques référents utilisés en aquaculture (enrofloxacine (C), érythromycine (D) et oxytétracycline (E). Elles sont testées par mesure des concentrations minimales inhibitrices exprimées en ppm vis-à-vis de 5 pathogènes aquatiques bactériens : *Aeromonas salmonicida salmonicida, Streptococcus iniae, Vibrio harveyi, Vibrio anguillarum, Vibrio parahaemolyticus*.

Après décongélation et repiquage des souches pathogènes, ces dernières sont cultivées sur milieu liquide Marine Broth en aérobie à 25°C pendant 24 heures. Le niveau de concentration bactérienne est variable selon les souches et atteint 10⁷ à 10⁹ UFC/mL après 24 heures. Ces souches pathogènes sont ensuite diluées pour atteindre une concentration de 10⁶ UFC/mL pour les tests de challenge.

Le mélange constitué des seuls composés R-SOa-S-R ou la composition constituée de l'ensemble des 8 ingrédients décrits dans l'invention ainsi que 3 antibiotiques cibles sont mis en solution dans de l'eau distillée et dilués successivement dans de l'eau distillée directement dans les puits d'une microplaque 96 puits. 100 µL de culture bactérienne cible à la dose de 10⁶ UFC/mL au moment de l'incubation sont ajoutés dans chaque puits de la microplaque. La microplaque est ensuite incubée 18h à 25°C. Les concentrations minimales inhibitrices sont ensuite calculées par observation des puits montrant ou non une croissance bactérienne en fonction de la dose de composition ou d'antibiotique associé. Les tests sont répétés au minimum 3 fois de suite afin d'obtenir une valeur moyenne robuste.

Après 18h d'incubation, les résultats (tableau 2 ci-après) indiquent que la composition à base des seuls composés R-SOa-S-R constituant 20 % de la composition de 8 ingrédients décrite dans l'invention présente des concentrations minimales inhibitrices similaires à la composition de 8 ingrédients. Les ingrédients de cette composition induisent donc une synergie renforçant le potentiel antimicrobien de la composition à base des seuls composés R-SOa-S-R et permettent de diminuer la dose de ces composés R-SOa-S-R dans la composition globale par un facteur 5 ce qui rend économiquement viable l'application de ladite composition.

Par ailleurs, après 18h d'incubation, la composition à base des seuls composés R-SOa-S-R ainsi que la composition de 8 ingrédients décrite dans l'invention présentent des concentrations minimales inhibitrices, comprises entre 19 et 150 ppm selon la souche, similaires (moins d'un facteur 10 de différence) à celles obtenues avec les antibiotiques : 3 à 9 ppm pour l'enrofloxacine, 19 à 300 ppm pour l'oxytétracycline et 25 et 75 ppm pour l'érythromycine.

**Tableau 2.** Le tableau 2 illustre les concentrations minimales inhibitrices (ppm) vis-à-vis de 5 souches pathogènes de la composition de 8 ingrédients décrite dans l'invention (A) en comparaison avec le mélange constitué des seuls composés R-SOa-S-R (B) et les 3 antibiotiques d'intérêt en aquaculture : enrofloxacine (C), érythromycine (D) et oxytétracycline (E).

| | **(A)** | **(B)** | **(C)** | **(D)** | **(E)** |
|---|---|---|---|---|---|
| ***Aeromonas salmonicida*** | 75 | 75 | 9,38 | 9,38 | >300 |
| ***Streptococcus iniae*** | 18,75 | 18,75 | 4,69 | <3,125 | 12,5 |
| ***Vibrio anguillarum*** | 25 | 18,75 | 3,12 | 50 | 18,75 |
| ***Vibrio harveyi*** | 100 | 150 | 6,25 | 50 | 37,5 |
| ***Vibrio parahaemolyticus*** | 50 | 37,5 | <3,12 | 18,75 | 12,5 |

### Exemple 4 - Test de l'efficacité de la prévention

Dans un premier temps, la composition décrite dans l'invention est testée en tant que méthode de prévention des maladies bactériennes en élevages aquacoles.

Elle est testée en conditions expérimentales d'élevage de bar européen (*Dicentrarchus labrax*), de tilapia du Nil (*Oreochromis niloticus*) et de crevette blanche (*Penaeus vannamei*), respectivement infectés par *Photobacterium damselae,* responsable de la pasteurellose chez le bar, *Streptococcus agalactiae* responsable de la streptococcose chez le tilapia et *Vibrio parahaemolyticus*, responsable de la nécrose hépatopancréatique aigue chez la crevette.

### A- Elevage de bars européens

Pour le bar européen (*Dicentrarchus labrax*), les juvéniles, pesant 8.7 ± 0.7 g sont répartis dans des bacs en fibre de verre de 200 L à raison de 35 individus par bac. L'eau de mer à une salinité de 35 ‰ et une température de 20±0.5°C circule en continu dans le système d'élevage. Une photopériode de 14h heures de jour/10 heures d'obscurité est respectée avec une oxygénation de l'eau maintenue à plus de 90%.

2 traitements sont appliqués : (A) un aliment spécifique pour le bar européen non supplémenté et (B) un aliment spécifique pour le bar européen supplémenté avec la composition appliquée en préventif à 0,1% de l'aliment. La dose de 0,1% correspond à l'estimation de la dose efficace nécessaire pour observer l'efficacité de la composition mentionnée dans les exemples 2 et 3.

Chacun des traitements est divisé en 3 réplicas. Le système étant en recirculation, une propagation du pathogène entre les bacs est envisageable et ne permet donc pas d'avoir un traitement sans poissons infectés.

Pendant toute la période de l'essai (28 jours sans infection suivi de l'infection puis de 21 jours de suivi post infection), les poissons sont nourris ad libitum 3 fois par jour (09:00, 14:00 et 17:00) pendant la semaine et 2 fois par jour (10:00 et 16:00) le weekend.

L'aliment spécifique au bar européen (A et B) est formulé de façon à couvrir les besoins nutritifs de l'espèce. Il contient donc 44 % de protéines et 17% de matières grasses. Chacun des aliments est façonné selon un processus bien identifié. Les ingrédients solides constituant l'aliment sont broyés dans un moulin marteau (Hosokawa Micron, SH1, Pays Bas) de façon à obtenir une mouture inférieure à 200 µm. Les poudres obtenues sont mélangées à la composition décrite dans l'invention (dans le cadre du premier traitement) et aux ingrédients liquides tels que les huiles dans un mélangeur à pales (Mainca RM90, Espagne). Le mélange est ensuite humidifié avec 25% d'eau. Les aliments sont ensuite produits par processus d'extrusion à température contrôlée au moyen d'un extrudeur double vis (Italplast P55, Italie). La taille finale des granulés d'aliment est de 1.5 mm. Suite au processus d'extrusion, les aliments sont séchés dans un four à convection (OP 750-UF, LTE Scientifics, Royaume Uni) à 40°C pendant 6 heures.

La composition décrite dans l'invention est ajoutée à l'aliment du traitement (B) au cours du processus d'extrusion à la dose de 0.1%.

Après 28 jours d'élevage dans les conditions décrites précédemment, les poissons de chacun des bacs sont infectés par *Photobacterium damselae subsp. piscicida* souche PP3. Après anesthésie suite à une immersion dans du 2-phenoxyetahnol à 200ppm, le pathogène est injecté par voie péritonéale avec 50µL de bouillon tryptique soja contenant 10³ UFC/mL de souche PP3. Une seule injection est réalisée.

Le suivi de mortalité se fait sur 21 jours. Les poissons morts sont quotidiennement retirés de chacun des bacs. Pendant cette période, les poissons continuent à être alimentés avec les mêmes traitements que décrits précédemment.

Les résultats de mortalité (**Figure 1**) indiquent que 21 jours après injection de la souche pathogène de *Photobacterium damselae subsp. piscicida* souche PP3, la composition décrite dans l'invention appliquée à 0,1% de l'aliment augmente significativement la résistance des juvéniles de bar européen au pathogène. La mortalité est ainsi réduite de 18,1% par rapport au traitement n'ayant pas reçu de composition. Cette même mortalité est également stabilisée 7 jours après injection dans le cas du traitement contenant la composition.

### B- Elevage de tilapia

Pour le tilapia du Nil, (*Oreochromis niloticus*), les juvéniles, pesant 12 g ± 0,4 sont répartis dans des bacs en plastique de 100 L à raison de 30 individus par bac. L'eau à une salinité de 0 ‰ et une température de 28,4±0.3°C est renouvelée en continu à raison de 200 l/h.

3 traitements sont appliqués : (A) un aliment spécifique pour le tilapia du Nil non supplémenté, (B) un aliment spécifique pour le bar européen supplémenté avec la composition appliquée en préventif à 0,1% de l'aliment et (C) un aliment spécifique pour le bar européen supplémenté avec un antibiotique (amoxycilline).

Chacun des traitements est divisé en 5 réplicas. Le système étant en recirculation, une propagation du pathogène entre les bacs est envisageable et ne permet donc pas d'avoir un traitement sans poissons infectés.

Pendant toute la période de l'essai (28 jours sans infection suivi de l'infection puis de 21 jours de suivi post infection), les poissons sont nourris ad libitum 3 fois par jour (09:00, 14:00 et 17:00) pendant la semaine et 2 fois par jour (10:00 et 16:00) le weekend.

L'aliment spécifique au tilapia du Nil (A, B et C) est formulé de façon à couvrir les besoins nutritifs de l'espèce. Il contient donc 30 % de protéines et 8% de matières grasses. Chacun des aliments est façonné selon un processus bien identifié. Les ingrédients solides constituant l'aliment sont broyés dans un moulin marteau (Hosokawa Micron, SH1, Pays Bas) de façon à obtenir une mouture inférieure à 200 µm. Les poudres obtenues sont mélangées à la composition décrites dans l'invention (dans le cadre du deuxième traitement) et aux ingrédients liquides tels que les huiles dans un mélangeur à pales (Mainca RM90, Espagne). Le mélange est ensuite humidifié avec 25% d'eau. Les aliments sont ensuite produits par processus d'extrusion à température contrôlée au moyen d'un extrudeur double vis (Italplast P55, Italie). La taille finale des granulés d'aliment est de 1.5 mm. Suite au processus d'extrusion, les aliments sont séchés dans un four à convection (OP 750-UF, LTE Scientifics, Royaume Uni) à 40°C pendant 6 heures.

La composition décrite dans l'invention est ajoutée à l'aliment du traitement (B) au cours du processus d'extrusion à la dose de 0.1%. L'antibiotique est quant à lui ajouté à l'aliment du traitement (C) au cours du processus d'extrusion à la dose de 80mg/kg de poids vif de poisson.

Après 28 jours d'élevage dans les conditions décrites précédemment, les poissons de chacun des bacs sont infectés par *Streptococcus agalactiae.*

L'infection expérimentale se fait par immersion directement dans les bacs d'élevage des poissons. Après coupure du système de recirculation, un volume fixe de 50 ml d'une culture de 24 heures de *Streptococcus agalactiae* de concentration 10⁹ UFC/mL est ajoutée à chaque bac de 100 L. Pendant 1 heure, les poissons restent en circuit fermé puis la recirculation est remise en route. L'infection progresse naturellement de poisson en poisson par simple contact. L'objectif du test d'infection est d'obtenir un minimum de 30% de mortalité sur la période de 3 semaines post infection. Un système d'éclairage ultraviolet entre chaque bac permet de limiter la diffusion des bactéries pathogènes et donc une contamination croisée.

Le suivi de mortalité se fait sur 21 jours. Les poissons morts sont quotidiennement retirés de chacun des bacs. Pendant cette période, les poissons continuent à être alimentés avec les mêmes traitements que décrits précédemment.

Les résultats de mortalité (**Figure 2**) indiquent que 21 jours après injection de la souche pathogène de *Streptococcus agalactiae,* la composition décrite dans l'invention appliquée à 0,1% de l'aliment augmente significativement la résistance des juvéniles de tilapia du Nil au pathogène. Les résultats indiquent également que la réduction de mortalité observée avec la composition est proche de celle observée avec l'antibiotique même si inférieure. La mortalité est ainsi réduite de 8.7 ± 4% par rapport au traitement n'ayant pas reçu de composition. L'amoxycilline quant à elle réduit la mortalité de 15.3 ± 7% par rapport au traitement n'ayant pas reçu de composition.

### C- Elevage de crevettes

Pour la crevette blanche (*Penaeus vannamei*), les post larves prises au stade 5 (PL5) sont réparties dans des aquariums en verre de 100 L chacun à raison de 1000 individus par aquarium. L'eau a une salinité de 25 ‰, une température de 27±1°C et est renouvelée quotidiennement à raison de 20% du volume total.

5 traitements sont appliqués : (A) un aliment spécifique pour la crevette blanche non supplémenté et n'étant pas amené à avoir une infection, (B) un aliment spécifique pour la crevette blanche non supplémenté et étant amené à avoir une infection, (C) un aliment spécifique pour la crevette blanche supplémenté avec la composition appliquée en préventif à 0,1% de l'aliment et étant amené à avoir une infection, (D) un aliment spécifique pour la crevette blanche supplémenté avec la composition appliquée en préventif à 0,3% de l'aliment et étant amené à avoir une infection et (E) un aliment spécifique pour la crevette blanche supplémenté avec la composition appliquée en préventif à 0,5% de l'aliment et étant amené à avoir une infection.

Pendant toute la période de l'essai (20 jours sans infection suivi de l'infection puis de 14 jours de suivi post infection), les post larves de crevettes sont nourries 5 fois par jour (07:00, 11:00, 15 :00, 19 :00 et 23:00).

L'aliment spécifique à la crevette blanche (A, B, C, D et E) est un aliment commercial adapté aux post larves de crevettes blanches sous forme de granulés pressés coulant de taille comprise entre 100 µm et 400 µm selon l'âge de la post larve.

La composition décrite dans l'invention est ajoutée à l'aliment du traitement (C) après le processus de presse des granulés à la dose de 0.1%. La composition décrite dans l'invention est ajoutée à l'aliment du traitement (D) après le processus de presse à la dose de 0.3%. La composition décrite dans l'invention est ajoutée à l'aliment du traitement (E) après le processus de presse à la dose de 0.5%. La quantité de la composition sous forme de poudre, équivalente à 0,1% ou 0.3% ou 0.5% de l'aliment est diluée dans de l'eau selon des rapports respectifs poudre:eau de 1:60, 1:20 et 1:12 puis pulvérisée directement à la surface de l'aliment grâce à une rampe de pulvérisation. Si besoin, les granulés sont ensuite enrobés avec de l'huile animale ou végétale afin d'éviter le relargage de la composition une fois dans l'eau d'élevage des crevettes.

Après 20 jours d'élevage dans les conditions décrites précédemment, les crevettes de chacun des bacs, excepté les bacs recevant le traitement (A) sont infectées par Vibrio parahaemolyticus, bactérie responsable de la nécrose hépatopancréatique aigüe chez la crevette.

L'infection expérimentale se fait par immersion directement dans les bacs d'élevage des crevettes. Une solution bactérienne de *Vibrio parahaemolyticus*, de concentration 10⁶ UFC/mL est ajoutée à chaque bac de 100 L. L'infection progresse naturellement de post larve en post larve par simple contact.

Le suivi de mortalité se fait sur 14 jours. Les crevettes mortes sont quotidiennement retirées de chacun des bacs. Pendant cette période, les crevettes continuent à être alimentées avec les mêmes traitements que décrits précédemment.

Les résultats de mortalité (**Figure 3**) indiquent que 14 jours après injection de la souche pathogène de *Vibrio parahaemolyticus*, la composition décrite dans l'invention appliquée à 0.1%, 0,3% ou 0,5% de l'aliment augmente significativement la résistance des post larves de crevettes blanches au pathogène. La mortalité est ainsi respectivement réduite de 42%, 54% et 57% par rapport au traitement infecté n'ayant pas reçu de composition. Les résultats de mortalité en fonction de la dose montrent également une réponse quadratique indiquant que même si 0,1% est une dose efficace, 0,5% n'est pas meilleure que 0,3% et cette dernière dose est donc considérée comme optimale en crevettes. Les crevettes non infectées quant à elles présentent une mortalité faible inférieure à 10% considérée comme naturelle dans ces conditions d'élevage.

### Exemple 6 - Etude physiologique

La composition décrite dans l'invention s'applique en tant que méthode de prévention des maladies bactériennes en élevages aquacoles. La prophylaxie du produit est due à l'activation des paramètres antimicrobiens du sang des poissons avant l'infection pathogénique ce qui permet aux poissons d'augmenter leur résistance au moment où le pathogène apparaît dans le milieu sans pour autant affecter sa formule sanguine de base.

Après 28 jours d'essai où les juvéniles de bar européen sont nourris soit avec (A) un aliment spécifique pour le bar européen non supplémenté et (B) un aliment spécifique pour le bar européen supplémenté avec la composition appliquée en préventif à 0,1% de l'aliment, 4 poissons par bac sont sélectionnés, puis anesthésiés dans 1000 ppm de 2-phenoxethanol. Des échantillons de sang sont alors prélevés. 2 aliquots de sang frais par bac sont homogénéisés pour la mesure des activités antimicrobiennes présentes dans le compartiment sanguin : activité lysozyme, explosion respiratoire, activité antimicrobienne et des paramètres hématologiques. 2 autres aliquots sont centrifugés à 8000 g pendant 10min à 4°C pour obtenir le plasma. Immédiatement après la collecte de sang, des frottis sont réalisés, séchés puis colorés avec un colorant de Wright (Hematocolor, Merck) puis fixés avec du formol-éthanol (10% formaldéhyde : 37 % éthanol absolu).

Le profil sanguin comporte les paramètres suivants : l'hématocrite qui correspond au volume occupé par les cellules rouges dans un volume global de sang, exprimé en %. La méthode consiste à remplir un tube capillaire avec du sang puis boucher une des extrémités du tube avec de fins morceaux de plasticine et centrifuger ensuite le tube pendant 10 min à 8000g. Après cela, le pourcentage relatif de cellules rouges sanguines (CRS) peut être calculé. L'hémoglobine (Hb) quant à elle est mesurée par méthode colorimétrique. A partir de ces 2 paramètres (CRS et HB), plusieurs autres paramètres sont calculés. Le volume corpusculaire moyen (VCM) correspond à la formule Hématocrite (%) / CRS (10⁶ µL)) × 10 et l'hémoglobine corpusculaire moyenne (HCM) correspond à la formule (Hémoglobine (g dl) / CRS (10⁶ µL)) × 10.

Le comptage des cellules blanches contenues dans le sang se fait par dilution au 1.20ème de sang homogénéisé dans une solution saline de Hank contenant de l'héparine (30 unités/mL). Le comptage des cellules rouges contenues dans le sang se fait quant à lui après dilution au 1.200 ème dans une solution saline de Hank contenant de l'héparine (30 unités/mL). Le comptage se fait ensuite sur une chambre de Neubauer. Les concentrations des cellules blanches et des cellules rouges sont respectivement exprimées en 10⁴ cellules/µL et 10⁶ cellules/µL.

Parmi les mécanismes immunitaires de défense de l'hôte, on observe la phagocytose qui s'accompagne d'une libération brusque de molécules réactives d'oxygène communément appelée explosion respiratoire. Elle est mesurée au niveau des leucocytes du sang des poissons prélevé à la fin des 28 jours d'essai. 4µL de sang frais sont dilués dans 96µL de solution saline de Hank contenant de l'héparine (30 unités/mL) et placés dans les puits d'une microplaque de 96 puits avec 100µL de suspension de luminol (2 mM de luminol dans 0,2 M de tampon borate à pH 9.0, avec 2 µg/mL d'acetate de mystirate de phorbol, Sigma) pendant 2 h à 25 °C. Chaque échantillon est comparé à un blanc où aucun échantillon de sang n'est ajouté. La chémi-luminescence produite par le luminol est mesurée toutes les 3 minutes grâce à un lecteur de luminescence (Synergy HT, Biotek) pour la génération de courbes cinétiques. Chaque échantillon est mesuré en 3 réplicas et la luminescence relative est exprimée en unités relatives de lumière (URL).

Le lysozyme est une molécule participant aux mécanismes de défense de l'hôte. Il est mesuré par un essai turbimétrique. Une solution de *Micrococcus lysodeikticus* (0,5 mg/mL dans une solution tampon phosphate 0,05M à pH 6.2) est préparée dans une microplaque. 15µL de plasma et 250µL de la solution décrite précédemment sont mélangés pour un volume final de 265µL. La réaction de lyse bactérienne se fait à 25°C, à une absorbance de 450 nm. Elle est lue après 0,5 et 4,5 min dans un lecteur de microplaques de Synergy HT. Une solution de lysozyme de blanc d'oeuf lyophilisé (Sigma) est diluée dans du tampon phosphate 0,05M à pH 6.2 afin d'établir une courbe étalon standard. Le taux de lysozyme des échantillons de sang est établi après utilisation de la formule extraite de la courbe étalon.

L'activité bactéricide contenue dans le sang des poissons est mesurée à l'aide de la souche Photobacterium damselae subsp. piscicida PP3. Les bactéries sont cultivées pendant 48 heures à 25°C sur de l'agar de soja tryptique (TSA; Difco Laboratoires) et sont ensuite inoculées sur du bouillon de soja tryptique (TSB; Difco Laboratoires), chacun des 2 milieux étant supplémenté par du NaCl à une concentration final de 1% (w/v). Les bactéries inoculées dans le bouillon sont ensuite cultivées pendant 24 h à 25°C et sous agitation constante à 100 tpm. Les bactéries en phase de croissance exponentielle sont récoltées par centrifugation à 3500 g pendant 30 min et ensuite resuspendues dans une solution saline de Hank contenant de l'héparine (30 unités/mL). Leur concentration est ajustée à 10⁶ UFC/mL. 20µL de plasma sont ajoutés dans les puits d'une microplaque de 96 puits. Une solution saline de Hank contenant de l'héparine (30 unités/mL) est utilisée comme contrôle. Dans chaque puits, 20 µL de souche PP3 à 10⁶ UFC/mL sont ajoutées puis incubés à 25°C pendant 2,5h. Dans chaque puits, 25µL de 3-(4,5 dimethyl-2-yl)-2,5-diphenyl bromide de tetrazolium (1 mg/mL, Sigma) sont ajoutés et incubés à 25°C pendant 10 min pour permettre la formation de formazan. Les microplaques sont ensuite centrifugées à 2000g pendant 10 min et le précipité est dissous dans 200µL de diméthyle de sulfoxide (Sigma). L'absorbance du formazan dissout est mesuré à 560 nm. L'activité bactéricide relative est exprimée en %, à partir de la différence entre les bactéries survivantes et le nombre de bactéries du contrôle.

Après 28 jours d'alimentation, la plupart des paramètres sanguins (voir tableau 4) concernant les cellules rouges sanguines et l'hémoglobine demeurent inchangés quelque soit le régime alimentaire (A ou B). La composition décrite dans l'invention appliquée à 0,1% de l'aliment ne modifie pas significativement les paramètres physiologiques de base. En revanche, la composition augmente les cellules blanches sanguines induites dans l'immunité même si cette augmentation n'est pas statistiquement significative. En parallèle, l'explosion respiratoire, le lysozyme et l'activité antimicrobienne des cellules blanches du sang sont significativement activées par la composition et leurs pourcentages d'augmentation par rapport au contrôle sont respectivement de 39%, 25% et 8%. Cette activation globale de la machinerie immunitaire innée renforce les poissons lors de l'apparition de pressions pathogéniques et permet de comprendre les mécanismes mis en jeu lors de l'application de la composition.

**Tableau 4**. Le tableau 4 illustre les paramètres sanguins et antimicrobiens observés chez les juvéniles de bar européen *Dicentrarchus labrax,* 28 jours après alimentation et avant infection avec *Photobacterium damselae subsp. piscicida* souche PP3 selon que les poissons sont non supplémentés (A) avec la composition décrite dans l'invention ou selon que cette dernière est appliquée à 0,1% (B)

| **Paramètres** | | **(A)** | **(B)** |
|---|---|---|---|
| **Hématocrite** | % | 22,6 | 19,3 |
| **Hémoglobine** | g/dl | 1,3 | 1 |
| **Volume Corpusculaire moyen** | µm³ | 120,7 | 108 |
| **Hémoglobine Corpusculaire moyenne** | pg/cellule | 6,7 | 5,8 |
| **Cellules rouges sanguines** | ×10⁶ µL | 1800000 | 1900000 |
| **Cellules blanches sanguines** | ×10⁴ µL | 47000 | 61000 |
| | | | |
| **Explosion respiratoire** | URL | 615 | 855 |
| **Lysozyme** | µg/mL | 2,8 | 3,5 |
| **Activité bactéricide** | % | 19 | 28 |

### Exemple 6 - Test de l'effet curatif

Dans un second temps, la composition décrite dans l'invention s'applique en tant que méthode de traitement des maladies bactériennes en élevages aquacoles.

Elle est testée en conditions naturelles d'élevage de tilapia du Nil (*Oreochromis niloticus*) infecté par *Streptococcus spp* responsable de la streptococcose.

Des juvéniles de tilapia du Nil ayant une taille uniforme et ne présentant pas de signes de maladies ou de malformations sont acclimatés pendant 4 semaines avec un aliment commercial de taille 1,5 mm jusqu'à ce qu'ils atteignent un poids initial de 10g par poisson en vue de l'essai commercial. Ces juvéniles sont ensuite distribués de manière homogène en fonction de leur poids dans 12 cages de 12m3 (2,5 x2,5 x2,0 m).

Suite à l'apparition d'une maladie bactérienne liée à *Steptococcus spp*, la composition décrite dans l'invention est appliquée selon 2 posologies : soit 8 jours, soit 14 jours.

3 traitements sont appliqués : (A) un aliment commercial spécifique pour le tilapia du Nil non supplémenté, (B) un aliment commercial spécifique pour le tilapia du Nil supplémenté avec la composition appliquée en curatif à 0,4% de l'aliment pendant 8 jours après apparition de la maladie et (C) un aliment commercial spécifique pour le tilapia du Nil supplémenté avec la composition appliquée en curatif à 0,4% de l'aliment pendant 14 jours après apparition de la maladie. Chaque traitement est appliqué sur 4 cages par traitement. Chaque cage contient 300 poissons.

De 10 à 200g de poids vif, les poissons sont nourris ad libitum 3 fois par jour (08:00, 12:00 et 17:00). De 200g à 600g de poids vif, les poissons sont nourris ad libitum 2 fois par jour (08:00 et 17:00)

L'aliment spécifique au tilapia du Nil (A, B et C) est un aliment aquacole tel que défini précédemment à l'exemple 1 et formulé de façon à couvrir les besoins nutritifs de l'espèce tout au long du cycle d'élevage.

Une fois la maladie déclarée, la composition décrite dans l'invention est ajoutée en curatif à l'aliment des traitements (B et C) directement sur les granulé extrudés et à la dose de 0.4%. La quantité de la composition sous forme de poudre, équivalente à 0,4% de l'aliment est diluée dans de l'eau selon un rapport poudre:eau de 1:15 puis pulvérisée directement à la surface de l'aliment grâce à une rampe de pulvérisation. Si besoin, les granulés sont ensuite enrobés avec de l'huile animale ou végétale afin d'éviter le relargage de la composition une fois dans l'eau d'élevage des poissons.

Suite à l'épisode infectieux lié à *Streptococcus spp* et l'application des compositions à 2 différentes posologies, les résultats de mortalité indiquent que l'application à 0,4% pendant 8 jours (B) induit une diminution de mortalité de 20% comparée au contrôle sans composition (A). Elle est de 40% pour le traitement ayant reçu la composition à 0,4% pendant 14 jours (C). Une meilleure croissance post maladie en termes de gain de poids et d'indice de conversion est également observée selon que la composition est appliquée pendant 8 jours (B) ou 14 jours (C). La biomasse finale respective des traitements B et C est ainsi augmentée de 55,2% et 117% par rapport à A; l'indice de conversion des traitements B et C est quant à lui optimisé de 32,5% et de 47,5% par rapport à A.

Les résultats (Tableau 3 ci-après) confirment que la composition décrite dans l'invention a un effet curatif après un minimum de 8 jours d'application mais que l'efficacité contre le pathogène est optimale après 14 jours d'application.

**Tableau 3.** Le tableau 3 illustre la mortalité et les paramètres de croissance (biomasse finale, kg et indice de conversion) observés chez les juvéniles de tilapia du Nil *Oreochromis niloticus,* après infection avec *Streptococcus spp* selon que les poissons sont non supplémentés (A) avec la composition décrite dans l'invention ou selon que cette dernière est appliquée à 0,4% pendant 8 jours (B) ou 14 jours (C) après apparition de la maladie

| | **(A)** | **(B)** | **(C)** |
|---|---|---|---|
| **Taux de survie (%)** | 30 | 50 | 70 |
| **Bomasse finale (kg)** | 210 | 326 | 456 |
| **Indice de conversion** | 4 | 2,7 | 2,1 |

L'invention n'est pas limitée aux modes de réalisations présentées et d'autres modes de réalisation apparaîtront clairement à l'homme du métier.

## Revendications

1. Composition comprenant au moins un composé de formule I suivante
R-SOa-S-R' (I)
dans lequel a est égal à 1 ou 2 et R et R' sont indépendamment l'un de l'autre un groupe alkyl en C₁ - C₅, linéaire ou branché, aliphatique ou non, pour une utilisation quotidienne ou plus régulière, pendant au moins huit jours, dans le cadre de la prévention des infections bactériennes, des animaux aquatiques élevés dans des structures aquacoles,
ledit au moins un composé susmentionné représentant 0,003 % en poids par rapport au poids total de la composition.

2. Composition pour son utilisation selon la revendication 1, ladite composition comprenant aux moins deux, notamment deux, composés de formule I distincte, lesdits au moins deux composés représentant 0,003% en poids par rapport au poids total de la composition.

3. Composition pour son utilisation selon la revendication 1 ou la revendication 2, dans laquelle ladite composition comprend un mélange des composés suivants
CH₃-CH₂-CH₂-SO-S-CH₂-CH₂-CH₃ et CH₃-CH₂-CH₂-SO₂-S-CH₂-CH₂-CH₃
ledit mélange représentant en poids 0,003% de ladite composition.

4. Composition pour son utilisation selon l'une quelconque des revendications 1 à 3, dans laquelle lesdits animaux de bassins d'aquaculture sont les poissons ou les crustacés décapodes.

5. Composition pour son utilisation selon l'une quelconque des revendications 2 à 4, dans laquelle les dits au moins deux composés dont dans des proportions relatives en poids variant de 60/40 à 40/60.

6. Composition comprenant au moins un composé de formule I suivante
R-SOa-S-R' (I)
dans lequel a est égal à 1 ou 2 et R et R' sont indépendamment l'un de l'autre un groupe alkyl en C₁C₅, linéaire ou branché, aliphatique ou non, pour une utilisation quotidienne ou plus régulière, pour une utilisation quotidienne ou plus régulière, pendant au moins huit jours, dans le cadre du traitement des infections parasitaires, et notamment bactériennes, des animaux aquatiques élevés dans des structures aquacoles,,
ledit au moins un composé susmentionné représentant de 0,009 à 0,012 % en poids par rapport au poids total de la composition.

7. Composition pour son utilisation selon la revendication 6, ladite composition comprenant aux moins deux, notamment deux, composés de formule I, ladite composition comprenant 0,009 à 0,012 % en poids desdits au moins deux, notamment deux, composés par rapport au poids total de la composition, ladite composition comprenant notamment un mélange des composés suivants
CH₃-CH₂-CH₂-SO-S-CH₂-CH₂-CH₃ et CH₃-CH₂-CH₂-SO₂-S-CH₂-CH₂-CH₃.

8. Produit alimentaire comprenant au moins un nutriment, et
- la composition telle que définie dans l'une quelconque des revendications 1 à 5, pour une utilisation selon l'une quelconque des revendications 1 à 5, ou
- la composition telle que définie dans l'une quelconque des revendications 6 à 7, pour une utilisation selon l'une quelconque des revendications 6 à 7.

9. Composition comprenant :
- au moins un composé de formule I suivante :
R-SOa-S-R' (I)
dans lequel a est égal à 1 ou 2 et R et R' sont indépendamment l'un de l'autre un groupe alkyl en C₁C₅, linéaire ou branché, aliphatique ou non, et
- au moins un support minéral, ledit au moins un composé représentant en poids 15 % par rapport au poids du mélange dudit au moins un composé et dudit au moins un support minéral.

10. Composition selon la revendication 9, comprenant :
a. 20% en poids du mélange dudit au moins un composé et dudit au moins un support minéral,
b. de 40 à 61 % en poids par rapport au poids total de la composition de composés permettant la stabilité et hydro dispersion de la composition, et
c. de 19% à 40 % en poids de composés immunostimulants.
